Europäisches Patentamt

European Patent Office

Office européen des brevets

(11) Publication number: **0 442 314 A2**

# EUROPEAN PATENT APPLICATION

(21) Application number: 91101059.3

(22) Date of filing: 28.01.91

(51) Int. Cl.⁵: **G01N 27/12, G01N 33/28**

(30) Priority: 02.10.90 JP 265872/90
29.01.90 JP 18701/90

(43) Date of publication of application:
21.08.91 Bulletin 91/34

(84) Designated Contracting States:
**DE FR GB IT**

(71) Applicant: **NIPPONDENSO CO., LTD.**
**1-1, Showa-cho**
**Kariya-city Aichi-pref. 448(JP)**

(72) Inventor: **Maeda, Yutaka**
**3-207, Takatsunami-cho**

Kariya-city, Aichi-pref.(JP)
Inventor: **Ueshima, Hiroshi**
**2-12-15, Misono-cho**
**Anjo-city, Aichi-pref.(JP)**
Inventor: **Nozawa, Masaei**
**114-1, Aramaki, Aza, Fukuoka-cho**
**Okazaki-city, Aichi-pref.(JP)**

(74) Representative: **Klingseisen, Franz, Dipl.-Ing.**
**et al**
**Dr. F. Zumstein Dipl.-Ing. F. Klingseisen**
**Bräuhausstrasse 4**
**W-8000 München 2(DE)**

(54) **An apparatus for detecting deterioration of the lubricating oil.**

(57) A membrane of a polyaniline which is treated with an electrical oxidation and an electrical reduction is used by immersing in a lubricating oil. An electrical resistance of the membrane is varied in accordance with the amount of acid which is produced by the deterioration of lubricating oil. A pair of metallic electrodes detects the change of the electric resistance of the membrane.

FIG.1

# AN APPARATUS FOR DETECTING DETERIORATION OF THE LUBRICATING OIL

## BACKGROUND OF THE INVENTION

The present invention relates to an apparatus for detecting deterioration of a lubricating oil used for an engine of an automobile or machine and etc.

The lubricating oil used for an engine of an automobile deteriorates in accordance with the running distance of the automobile. The lubricating oil has a lubricating function, cooling function, sealing function, anti-corrosive function, and etc. Since these functions decline in accordance with the deterioration of the lubricating oil, the lubricating oil should be changed within a good interval. The speed of deterioration of the lubricating oil is not constant but varied in accordance with different conditions, for example, it depends on the quality of the lubricating oil, the model of an engine, the driving situation, the characteristic of the fuel, and the way of maintenance etc. The lubricating oil is wasted when the change of the lubricating oil is too early, on the other hand, the engine is damaged when the change of the lubricating oil is too late.

To detect the deterioration of lubricating oil, an apparatus using optical characteristic (transmittance), an apparatus using electrical characteristic (Japan utility patent laid open publication 59-3360. It is also known in Japan laid open publication 57-118153 that a pH sensor comprising a conducting body is covered by a membrane of polymer induced from a hydroxy aromatic compound. Japan patent laid open publication 56-47614 shows the deterioration detecting way carried by immersing a pH sensor into the lubricating oil directly.

The apparatus using an optical characteristic or the apparatus described in Japan utility patent laid open publication 59- 3360 have a defect that is hard to be detected the deterioration of the lubricating oil correctly because of the transmittance, and that the electrical resistance or capacitance doesn't have a correlation relation with the deterioration of the lubricating oil. In the Japan laid open patent 57-118153, the way to measure the deterioration of the lubricating oil is not described. Furthermore, in the Japan laid open patent 56-47614, it is impossible to measure pH directly since acid molecules are not dissociated into ions in the lubricating oil for the automobile.

## SUMMARY OF THE INVENTION

An object of the present invention is to provide an apparatus for detecting deterioration of the lubricating oil which can detect the deterioration of the lubricating oil correctly.

Another object of the present invention is to provide an apparatus for detecting deterioration of the lubricating oil so that an adequate changing time of the lubricating oil can be noticed.

In the present invention, a membrane of a conducting polymer treated with an electrical oxidation and an electrical reduction is used. An electrical resistance of the membrane changes in accordance with the amount of acid which is produced by the deterioration of the lubricating oil. An electrical resistance detecting means detects the change of the electrical resistance of the membrane. Since the membrane is treated with an electrical oxidation and an electrical reduction, the electrical resistance of the membrane changes linearly according to a total acid value of the lubricating oil.

## BRIEF DESCRIPTION OF THE DRAWINGS

Fig. 1 is a perspective view of a main part of the sensor of the first embodiment;

Fig. 2 is a cross sectional view of the sensor of Fig. 1;

Fig. 3 is a schematic view showing the apparatus of the first embodiment;

Fig. 4 is an electric circuit showing a signal conditioning circuit used in the apparatus of the first embodiment;

Fig. 5 is a schematic view showing the apparatus of the first embodiment equipped to an automobile;

Fig. 6 is a graph showing the relation between an electrical resistance and an increased total acid value under the condition without a reduction in a perchloric acid aqueous solution;

Fig. 7 is a graph showing the relation between an electrical resistance and an increased total acid value under the condition of a reduction in a perchloric acid aqueous solution shown in the first embodiment of the present invention;

Fig. 8 is a graph showing the relation between an electrical resistance and the used time of the detecting apparatus without adding polyanion to the membrane in the first embodiment of the present invention;

Fig. 9 is a graph showing the relation between the electrical resistance and the used time of the detecting apparatus with adding polyanion to the membrane in the first embodiment of the present invention;

Fig. 10 is a plain view showing the detecting apparatus of the second embodiment of the present invention;

Fig. 11 is a cross sectional view showing the detecting device of the third embodiment of the present invention;

Fig. 12 is a cross sectional view of the detecting apparatus of the forth embodiment of the present invention;

Fig. 13 is a schematic view of the detecting apparatus of the fifth embodiment of the present invention;

Fig. 14 is a graph showing the relation between pH and the increased total acid value of the fifth embodiment of the present invention;

Fig. 15 is a schematic view showing the detecting apparatus of the sixth embodiment of the present invention;

Fig. 16 is sectional view of a part of the detecting apparatus shown in Fig. 15.

DETAILED DESCRIPTION OF THE PREFERRED EMBODIMENTS

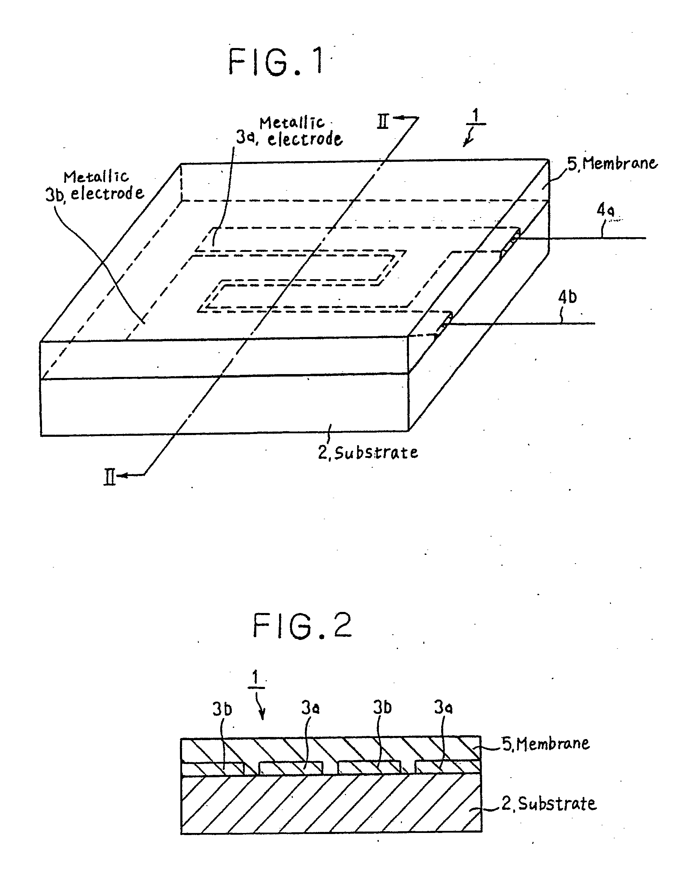

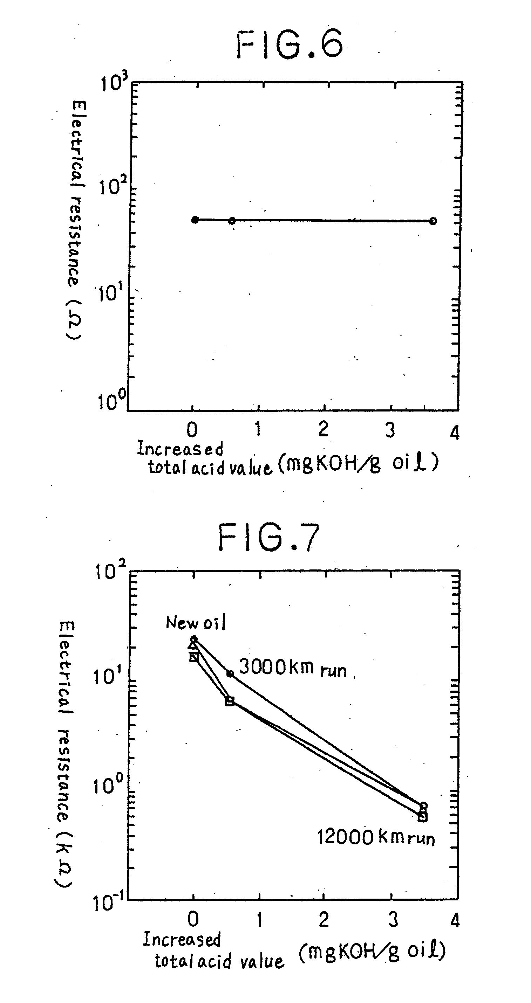

The apparatus of the first embodiment of the present invention is described hereafter based on Fig. 1 to Fig. 9. A detecting device 1 is shown in Fig. 1 and Fig. 2. On a ceramic insulating substrate 2, a pair of metallic electrodes 3a and 3b having C shape portion are formed. Both of the metallic electrode 3a and 3b face each other. Leads 4a and 4b are connected to the ends of the metallic electrodes 3a and 3b respectively. A membrane of a conducting polymer is formed on the metallic electrodes 3a and 3b. This membrane of a conducting polymer is produced by the potentiostatic electrolysis polymerization at 1200mV using a silver electrode and a silver chloride electrode. The potentiostatic electrolysis polymerization is carried out in a perchloric acid aqueous solution of 0.5mol/l including aniline of 0.2mol/l and a sodium polystyrene sulfonate of 5wt% as a polyanion. The molecular weight of the sodium polystyrene sulfonate is $10^5$. Furthermore, an electrical reduction is carried out on the membrane for 5 minutes with -50mV in the perchloric acid aqueous solution of 0.5mol/l. The 5 minutes impression of the voltage of -50mV is carried out by using the silver electrode and the silver chloride electrode. Then, an electrical oxidation is carried out on the membrane for 5 minutes with 500mV in an alkali aqueous solution (pH 11.0) including a sodium sulfate of 0.5mol/l. This 5 minutes impression of the voltage is also carried out by using the silver electrode and the silver chloride electrode. Then, an electrochemical reduction is carried out for 5 minutes with -250mV using the silver electrode and the silver chloride electrode. After all of these processes, a membrane 5 of polyaniline is formed. If there is no electrical reduction process in the perchloric acid aqueous solution, there is no sensitivity against the total acid value of the lubricating oil as shown in Fig. 6. After carrying out the electrical reduction in the perchloric acid aqueous solution, the conductivity changes exponentially against the total acid value of the lubricating oil as shown in Fig. 7. The graphs of Figs. 6 and 7, are drawn by using 3 data.

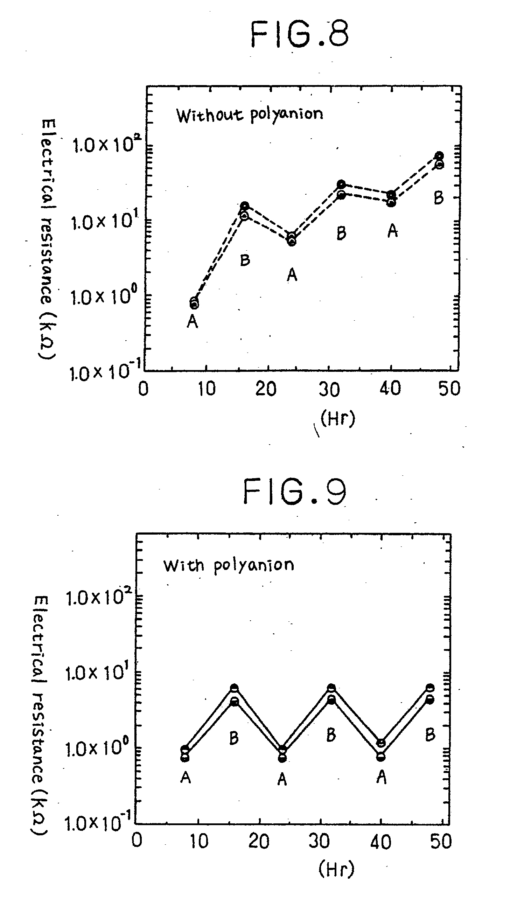

If there is no sodium polystyrene sulfonate as a polyanion, the repeatability of the membrane 5 becomes worse as shown in Fig. 8. In this Fig. 8, a degree of the deterioration of the membrane 5 is measured in the lubricating oil at 130°C, and the relation between the electrical resistance of the membrane 5 and the used time is shown. The electrical resistance increases as in accordance with a used time of the membrane 5. In Figs. 8 and 9, A represents a deteriorated oil and B represents a changed fresh oil.

After adding polyanion as described above, the repeatability of the membrane 5 becomes good as shown in fig. 9. In other words, the life on the deterioration detecting apparatus becomes long when a polyanion is added.

In this first embodiment, other polymers which includes nitrogen atom like poly-pyrrole, poly-N-alkyl pyrrole, or poly-N-arylpyrrole etc. can be used instead of polyaniline. The reason of the characteristic described above is considered that a proton of the organic acid coordinates to an independent electron-pair of the nitrogen atom.

In the first embodiment, other acids comprising polyanion whose molecular weight is over $10^3$ or alkali metal salt can be used. For example sodium polyvinyl sulfonate($(CH_2CHSO_3Na)n$), sodium polyacrylate $((CH_2CHCOONa)n)$, sodium polyphosphate($(NaH_5P_4O_{13})n$), phosphomolybdic acid($Na_3(PO_4/12M_0O_3)$), or phosphotungstic acid-($Na_3(PO_4 \cdot 12WO_3)$) etc. can be used instead of sodium polystyrene sulfonate $((CH_2CH-(C_6H_4SO_3Na))n)$.

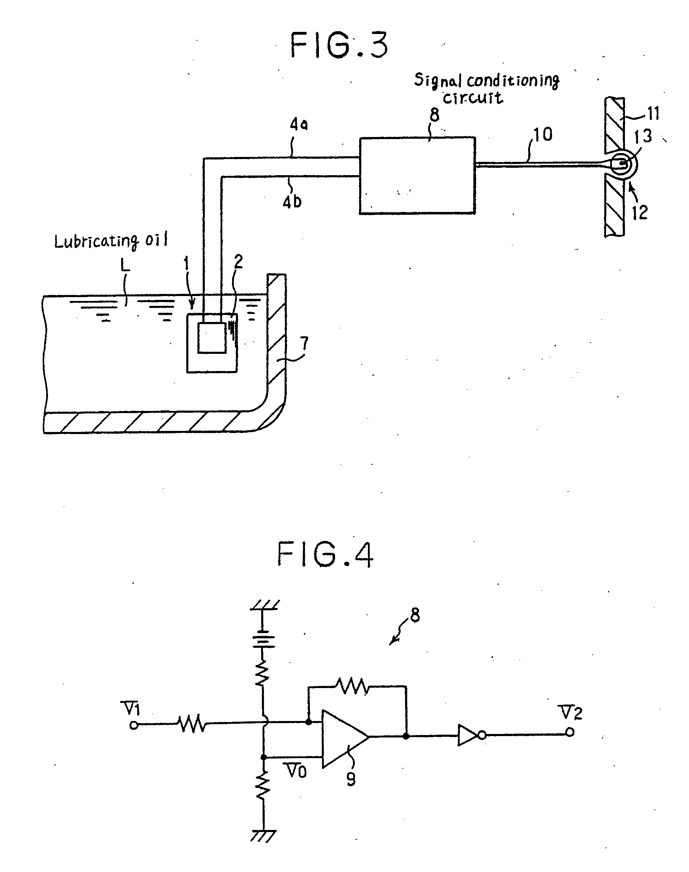

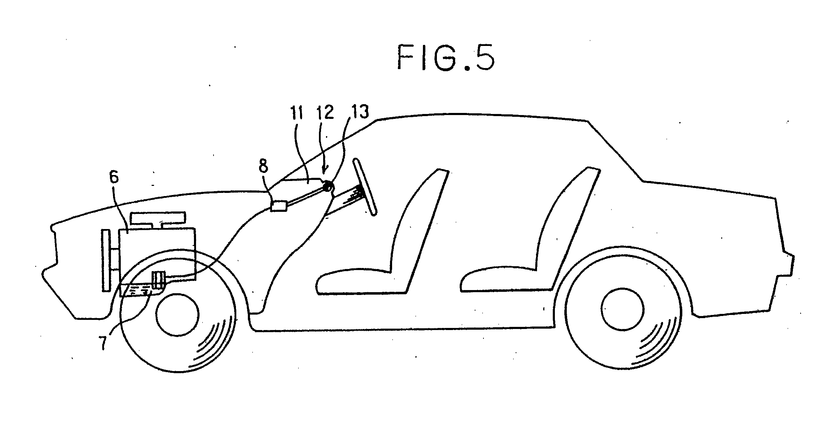

In the first embodiment of the present invention, the preferable amount of sodium polystyrene sulfonate is over 5wt%. The desirable molecular weight of the sodium polystyrene sulfonate is $10^5$. The polyanion is considered to be taken into the membrane of the polyaniline as a dopant at the polymerization. The polyanion is considered not to be undoped from the membrane of the polyaniline because of the large number of molecular weight when it is used in the lubricating oil. The detecting device 1 is immersed into the lubricating oil 1 in an oil pan 7 located below an automobile engine 6 as shown in Fig. 3 and Fig.5. The leads 4a and 4b are connected to a signal conditioning circuit 8. As shown in Fig. 4, the output voltage $V_1$ according to the change of the electrical resistance of the detecting device 1 is input to the comparater 9. After comparing the voltage $V_1$ and the predetermined voltage $V_0$, a voltage $V_2$ is output when the voltage $V_1$ is smaller than the predetermined voltage $V_0$.

As shown in Fig. 3, the signal conditioning circuit 8 is connected to a display lump 13 equipped in a instrumental panel 11 inside the car by leads 10. The display lump 13 is a part of an warning apparatus 12. The warning apparatus 12 can warn to the driver by displaying the deterioration of the lubricating oil on the display lump 13 according to the output voltage $V_2$ from the signal conditioning circuit 8.

As shown in Fig. 7, the electrical resistance of the detecting device 1 decreases exponentially against the increased total acid value which is the amount of the total acid produced by the deterioration of the lubricating oil.

The lubricating oil in the oil pan 7 deteriorates gradually by the air or heat, and the organic acid is produced. The electrical resistance of the membrane 5 made of polyaniline decreases gradually by the organic acid caused by the deterioration of the lubricating oil. It means that the voltage between electrodes 3a and 3b is decreased. Since the membrane of the polyaniline formed on the electrodes 3a and 3b is very thin, for example, the thickness is several $\mu$m, the response time is quite short.

In the first embodiment, the signal conditioning circuit 8 as shown in fig. 4 is used as the electrical resistance detecting means, but a measurement of an alternating-current resistance and an alternating-current impedance can be used instead of the signal conditioning circuit 8 shown in fig. 4.

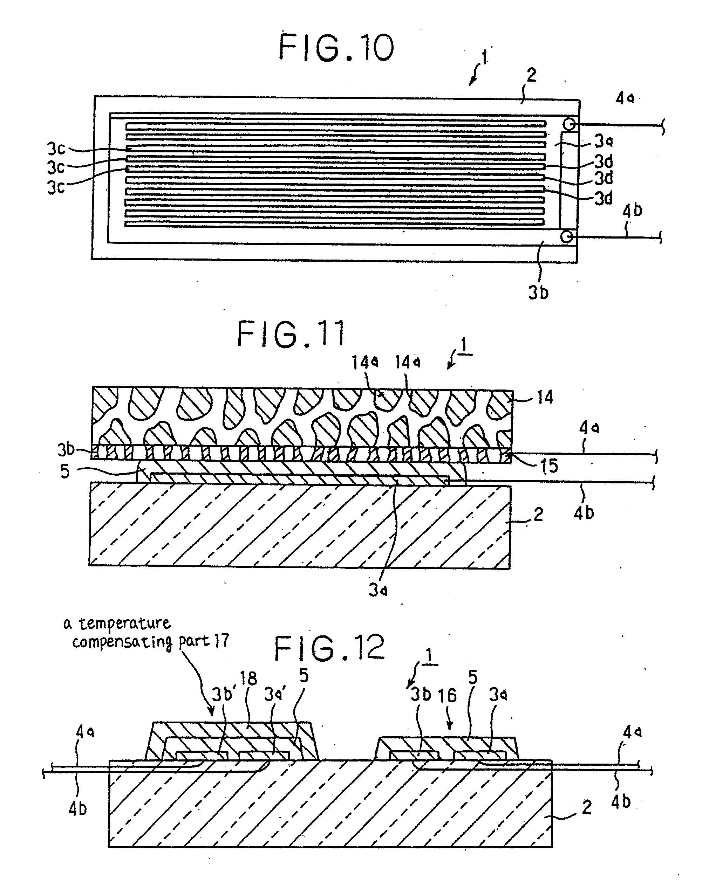

The second embodiment of the present invention is described by using fig. 10. Most parts of the second embodiment are the same as the first embodiment except the shape of metallic electrodes. As shown in fig. 10, a pair of metallic electrodes 3a and 3b are formed on the ceramic insulating substrate 2. The metallic electrodes 3a and 3b have comb shaped parts 3c and 3d respectively. The width of the comb shaped part 3c and 3d is about few $\mu$m, the comb shaped part 3c faces to comb shaped part 3d like a pair of gears. According to this construction, the detecting device 1 can be miniaturized because the lowered electrical resistance of the membrane caused by the long facing parts between the electrode 3a and the electrode 3b. The membrane 5 made of polyaniline can be thin according to the second embodiment of the present invention. The electrical resistance of the membrane 5 is changed since the ion exchange between polyaniline and the organic acid molecules through the boundary between the membrane 5 and the lubricating oil L. Very thin membrane is required to make the reaction (ion exchange) faster. At this point, the second embodiment can achieve the quick response time.

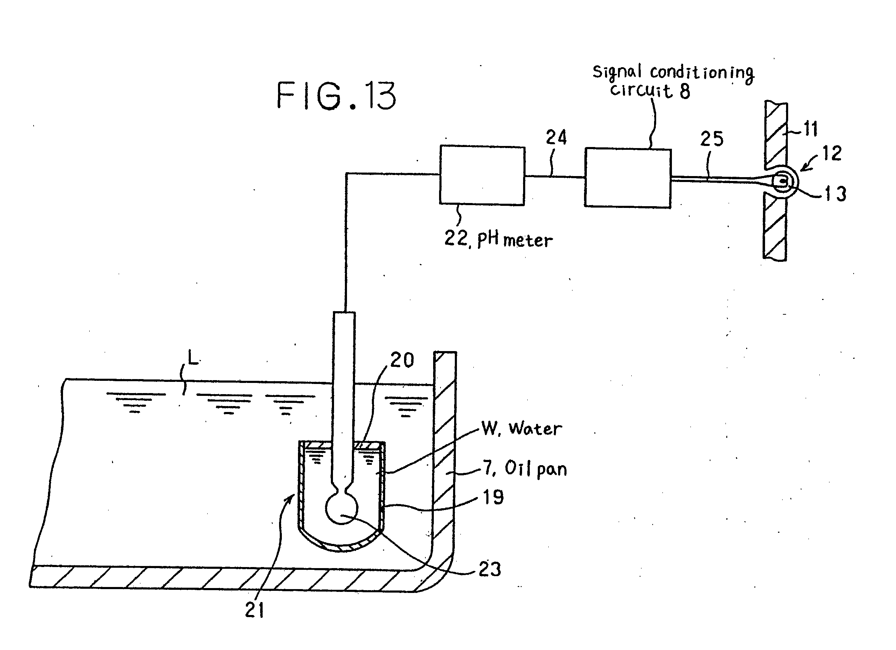

The third embodiment of the present invention is shown in Fig. 11. In the third embodiment, the same numerals represent the same parts described in the first embodiment. The metallic electrode 3a which is formed by platinum, gold, or silver by using the vacuum evaporation on the glass substrate 2. The membrane 5 of polyaniline is formed on the metallic electrode 3a by the potentiostatic electrolysis polymerization. The thickness of the membrane 5 is under few $\mu$m. Porous support 14 is made of ceramic or nonwoven fabric. Beneath the porous support 14, a porous electrode 15 which is made by vacuum evaporation using a platinum, gold, or silver. Then, the porous electrode 15 and the membrane 5 are attached. The substrate 2 and the metallic electrode 3a can be porous.

According to the third embodiment, the lubricating oil L reaches to the membrane 5 of polyaniline from the top portion in Fig. 11 through pores 14a of the porous support 14 and pores of the porous electrode 15.

The forth embodiment of the present invention is shown in Fig. 12. In this embodiment, a temperature compensating part 17 is different from the first embodiment. The same numerals represent the same parts described in the first embodiment. A pair of metallic electrodes 3a and 3b are formed on the glass substrate 2 by the vacuum evaporation. The membrane 5 of polyaniline is formed on the metallic electrodes 3a and 3b. A covering layer 18 is formed on the membrane 5 at the temperature compensating part 17. This covering layer 18 is made of an epoxy resin, or silicon rubber and has an oil-resistant characteristic and a heat-resistant characteristic. The covering layer 18 prohibits the organic acid from the self transmitting. At the detecting part 16, the voltage between the electrode 3a and the electrode 3b decreases according to the organic acid produced in the lubricating oil L as described in the first embodiment. On the other hand, at the temperature compensating part 17, there is no change of the electrical resistance because of the covering layer 18. Both temperatures of the membrane 5 at the detecting part 16 and the membrane 5 at the temperature compensating part 17 are changed according to the temperature of the lubricating oil L. Then, the temperature compensation can be achieved because of the difference of the electrical resistance between the detecting part 16 and the temperature compensating 17.

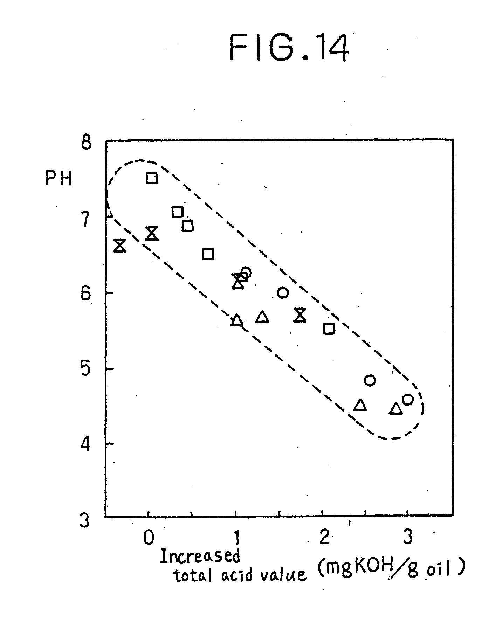

The fifth embodiment of the present invention is shown in Fig. 13 and Fig. 14. In the fifth embodiment, the same numerals represent the same parts described in the first embodiment. In the lubricating oil L, a case 21 is filled with the water W as a hydric medium. This case 21 comprises a cap 20 and a silindrical porous membrane 19 with a bottom. A hydrophilic cellulose ester, for example acetylcellulose or ethylcellulose can be used as the porous membrane 19. The preferable diameter of the pore is around 0.2 $\mu$m. The organic acid can not transmit the porous membrane 19 when the diameter of the pore is too small. On the other hand, the lubricating oil L and the water W are mixed together when the diameter of the pore is too large. An isopropyl alcohol, a butyl alcohol and other alcohols can be used as a hydric medium instead of the water W. In the water W, a pair of electrodes 23 covered by a conducting polyaniline are immersed in the water W. A pair of electrode 23 are connected to a pH meter 22. The pH meter 22 is connected to the indicating lump 13 through the signal conditioning circuit 8. It is better to cool the lubricating oil L in the oil pan 7 below 80°C to protect the ebullition of water W.

As shown in Fig. 14, the pH of water W decreases linearly when the amount of the organic acid increases according to the deterioration of the lubricating oil L in the oil pan 7. The total acid value is measured by JIS(Japanese Industrial Standard) K2501. Symbols O, □ and etc. represent the kinds of the lubricating oil L in Fig. 14.

When the lubricating oil L is used repeatedly, the organic acid is produced because of the deterioration of the lubricating oil L by heat or air. The organic acid transmits the porous membrane 19 and changes the aqueous solution into acid by dissolution into the water W. The change of the aqueous solution into the acid is detected by the composite electrode 23 connected to the pH meter 22. The detected signal is sent to the signal conditioning circuit 8, and the same processes described in the first embodiment are carried out to warn the deterioration of the lubricating oil L to the driver.

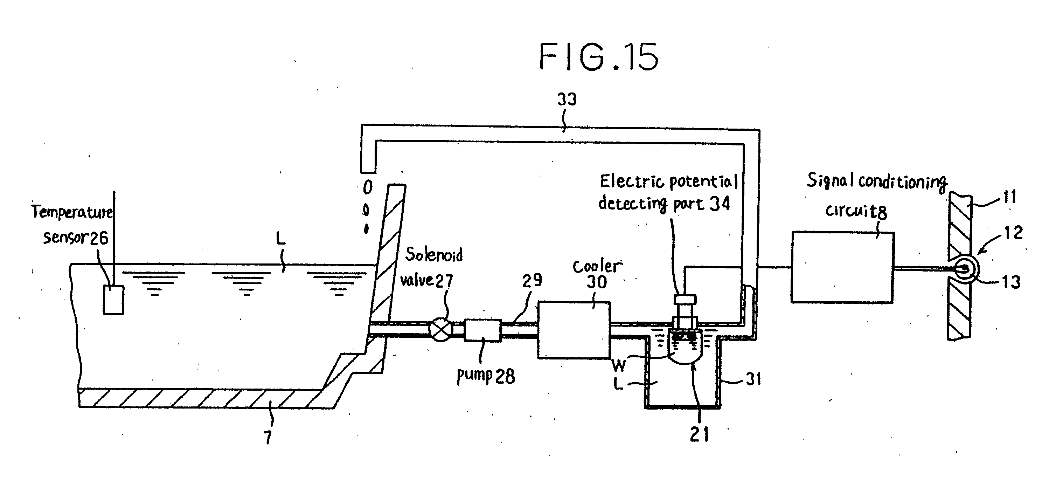

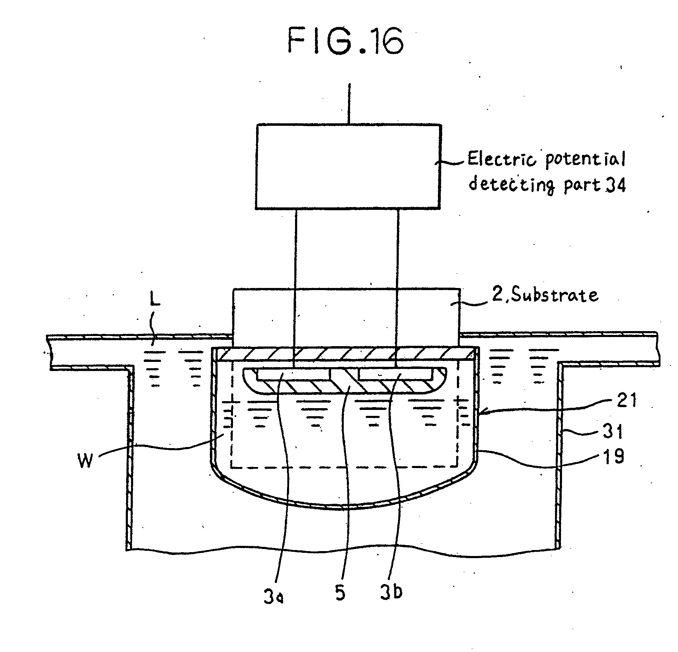

The sixth embodiment of the present invention is shown in Fig. 15 and Fig. 16. In the sixth embodiment, the most important point is to cool the lubricating oil around the membrane 5 of polyaniline. A temperature sensor 26 is immersed in the lubricating oil L. The lubricating oil L in the oil pan 7 is led to a tube 29 through a solenoid valve 27 and a pump 28. The tube 29 is connected to a cooler 30 for cooling the lubricating oil before detecting an electric potential of the detecting device 1. The solenoid valve 27 opens the tube 29 when the temperature of the lubricating oil is lower than the predetermined temperature. When the solenoid valve 27 is opened, the lubricating oil L is led to the cooler 30 by the pump 28. The lubricating oil cooled by the cooler 30 flows into the receiver 31 and returns to the oil pan 7. The case 21 is the same one described in the previous embodiments. The detecting device 1 comprises a pair of electrodes 3a and 3b covered by the membrane 5 of polyaniline the thickness of which is about 10$\mu$m. The lubricating oil returns to the oil pan 7 through a circulation tube 33.

In this sixth embodiment, the organic acid produced by the deterioration of the lubricating oil L transmits the porous membrane 19 and goes into the case 21. The electrical resistance of the membrane 5 of polyaniline is decreased when the organic acid resolves into the water W in the case 21. Then, the electric potential between the electrode 3a and the electrode 3b is also decreased. The decreased electric potential is detected by the electric potential detecting part 34, and the detected signal is calculated by the signal conditioning circuit 8. When the electric potential is lower than the predetermined electric potential, the indicating lump 13 is lightened.

In all embodiments described above, the detecting device 1 can be used with a pressure sensor or a temperature sensor as one sensing device to get the informations of lubricating oil, like a temperature of the oil, a pressure of the oil, a deterioration of the oil and etc. by one sensing device.

The detected signal detected by a pair of electrodes 3a and 3b can drive any controlling units, for example, a controlling unit for control the adding amount of the anti-deterioration.

This apparatus for detecting deterioration of the lubricating oil can be used not only for the car but also for other machines.

**Claims**

1. An apparatus for detecting deterioration of a lubricating oil electrically, characterized in that said apparatus further comprises;
   a membrane of a conducting polymer treated with an electrical oxidation and an electrical reduction, wherein an electrical resistance of said membrane is changed according to acid which is produced by the deterioration of said lubricating oil, and
   an electrical resistance detecting means for detecting a change of said electrical resistance of said membrane.

2. An apparatus for detecting deterioration of a lubricating oil according to claim 1;
   wherein said membrane comprises a polyaniline.

3. An apparatus for detecting deterioration of a lubricating oil according to claim 1 or 2,
   wherein said membrane includes a polyanion.

4. An apparatus for detecting deterioration of a lubricating oil according to claims 1 to 3,
   wherein said membrane includes a nitrogen atom.

5. An apparatus for detecting deterioration of a lubricating oil according to preceding claims;

   wherein said electrical resistance detecting means comprises a pair of comb shaped electrodes.

6. An apparatus for detecting deterioration of a lubricating oil according to preceding claims, further comprising;

   a temperature compensating means for compensating a change of said electrical resistance caused by a change of temperature.

7. An apparatus for detecting deterioration of a lubricating oil according to preceding claims, further comprising;

   a cooling means for cooling said lubricating oil.

# FIG.1

Metallic 3a, electrode

Metallic 3b, electrode

II

1

5, Membrane

4a

4b

2, Substrate

# FIG.2

1

3b  3a  3b  3a

5, Membrane

2, Substrate

# FIG.3

Signal conditioning circuit

8

4a

4b

Lubricating oil

L

1    2

7

10

11

13

12

# FIG.4

8

$V_1$

$V_0$

9

$V_2$

FIG.5

# FIG.6

Y-axis: Electrical resistance ($\Omega$)
X-axis: Increased total acid value (mgKOH/g oil)

# FIG.7

Y-axis: Electrical resistance ($k\Omega$)
X-axis: Increased total acid value (mgKOH/g oil)

New oil
3000km run
12000 km run

# FIG.8

# FIG.9

EP 0 442 314 A2

# FIG.10

# FIG.11

# FIG.12

a temperature compensating part 17

## FIG.13

Signal conditioning circuit 8

24

25

11

12

13

22, PH meter

L

20

W, Water

7, Oil pan

19

23

21

EP 0 442 314 A2

# FIG.14

# FIG.15

Temperature sensor 26

Electric potential detecting part 34

Signal conditioning circuit 8

Solenoid valve 27

pump 28

Cooler 30

21

31

33

7

L

W L

11

12

13

EP 0 442 314 A2

# FIG.16

Electric potential detecting part 34

2, Substrate

L

21

31

19

W

3a    5    3b